Europäisches Patentamt

European Patent Office

Office européen des brevets

(11) Publication number: **0 462 291 A1**

## EUROPEAN PATENT APPLICATION
### published in accordance with Art. 158(3) EPC

(21) Application number: **91901903.4**

(22) Date of filing: **09.01.91**

(86) International application number:
**PCT/JP91/00008**

(87) International publication number:
**WO 91/10371 (25.07.91 91/17)**

(51) Int. Cl.⁵: **A23L 1/30, A61K 37/22, C07G 17/00, C12N 9/99**

(30) Priority: **09.01.90 JP 1002/90**

(43) Date of publication of application:
**27.12.91 Bulletin 91/52**

(84) Designated Contracting States:
**CH DE FR GB IT LI NL SE**

(71) Applicant: **KABUSHIKI KAISYA ADVANCE KAIHATSU KENKYUJO**
**5-7 Nihonbashi Kobuna-cho**
**Chuo-ku Tokyo 103(JP)**

(72) Inventor: **ISHIHARA, Kazuoki**
**4-4-12-1302, Sagamihara Sagamihara-shi Kanagawa 229(JP)**
Inventor: **SHIN, Ryouichi**
**Haimunagara 103 6-2-14, Sagamihara Sagamihara-shi Kanagawa 229(JP)**

(74) Representative: **Rinuy, Santarelli**
**14, avenue de la Grande Armée**
**F-75017 Paris(FR)**

(54) **ACAT ENZYME INHIBITING COMPOSITION.**

(57) An ACAT enzyme inhibiting composition containing as an active ingredient a lipid acid-like substance originating in a microorganism or a plant lipid and having a molecular formula of $C_{18}H_{32}O_2$ or $C_{18}H_{30}O_2$.

Fig. 1

## TECHNICAL FIELD

The present invention relates to a novel ACAT enzyme inhibitory composition, more specifically to a lipid absorption inhibitory edible composition containing an ACAT enzyme inhibitory agent derived from a food component.

## BACKGROUND ART

In the absorption of a lipid, particularly cholesterol, a control of the rate of change of a free cholesterol to an ester cholesterol through an ACAT (AcylCoA-Cholesterolacyltransferase) enzyme in small intestine epithelial cells is known, and the usefulness of an ACAT inhibitory agent as the anti-cholesterol agent has been investigated, and currently several such agents have been developed as pharmaceuticals.

## DISCLOSURE OF INVENTION

Accordingly, an object of the present invention is to develop an ACAT enzyme inhibitory composition derived from a food component.

Other objects and specific features of the present invention will be apparent from the following description.

In accordance with the present invention, there is provided an ACAT enzyme inhibitory composition which is derived from a microorganism or vegetable lipid and contains a lipid acid-like substance having the formula of $C_{18}H_{32}O_2$ or $C_{18}H_{30}O_2$ as the active ingredient.

## BRIEF DESCRIPTION OF DRAWINGS

Figure 1 is an MS spectrum chart of a fatty acid-like substance A.

Fig. 2 is an MS spectrum chart of the methylesterified product of a fatty acid-like substance A.

Fig. 3 is an MS spectrum chart of a fatty acid-like substance B.

Fig. 4 is an MS spectrum chart of the methylesterified product of a fatty acid-like substance B.

## BEST MODE FOR CARRYING OUT THE INVENTION

The present inventors carried out intensive studies in order to accomplish the above object, and consequently found that a certain kind of fatty acid-like substance derived from microorganism lipids such as lactic acid bacteria and decomposed products of rice bran and wheat bran, which are suitable for common use as a functional food component, have a remarkable enzyme inhibitory activity.

The present invention is now described in detail as follows.

## FATTY ACID-LIKE SUBSTANCE

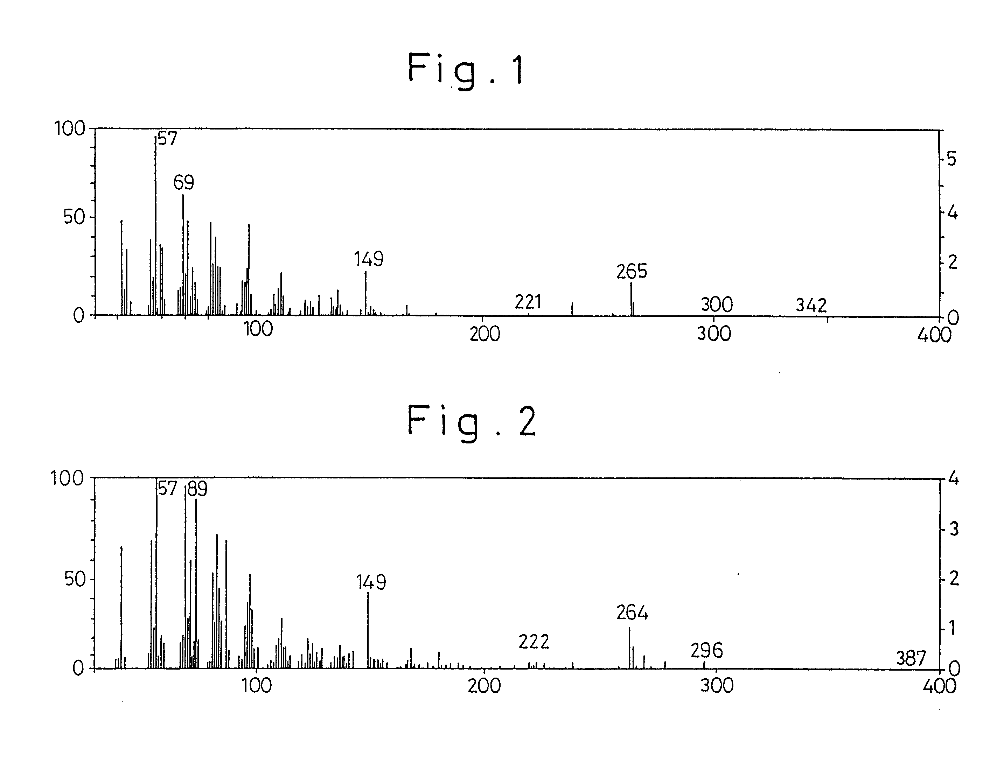

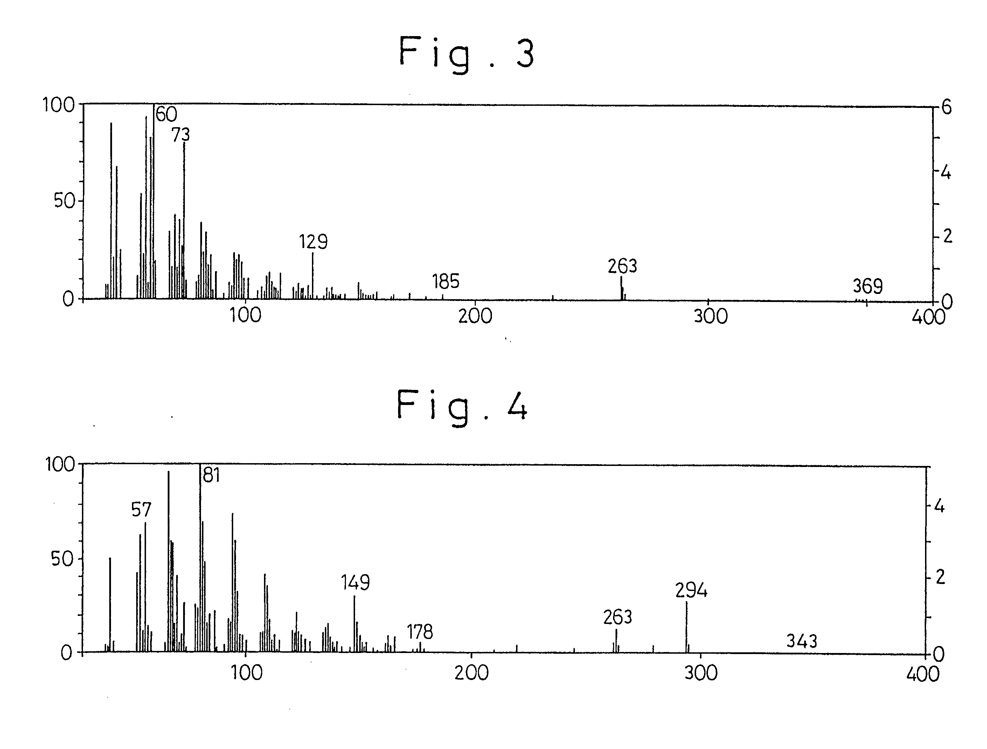

The fatty acid-like substance according to the present invention exists in microorganism lipids of lactic acid bacteria, and hydrolyzed (enzyme) decomposed products of vegetable lipids such as rice bran and wheat bran lipids, and the molecular formula thereof when purified and isolated in a conventional manner is $C_{18}H_{32}O_2$ or $C_{18}H_{30}O_2$ (Fig. 1 - Fig. 4: see MS spectrum charts of the respective substances and methylesterified products thereof), exhibiting fatty acid-like properties.

The ACAT enzyme inhibitory composition according to the present invention can be prepared by formulating these fatty acid-like substances in an appropriate carrier which will facilitate an oral administration thereof, for example, hydroxypropylmethyl cellulose phthalate and casein, (preferable content, 0.5 to 3% by weight), and further formulating, if necessary, a perfume or dye, etc.

## METHOD OF USE AND DOSAGE

The above substances usually can be administered orally in a dosage form such as a tablet or capsule, at a dosage of about 0.1 mg to 10 mg/kg (body weight)/day, and as food components, and therefore, are substantially orally nontoxic.

Also, when provided for utilization in an edible composition form, these substance need not be isolated for utilization, and may be utilized as food auxiliary components as a microorganism lipid, rice bran, or wheat bran lipid decomposed product, as such.

## ACAT ACTIVITY ASSAYING METHOD

This is practiced according to the method of Heider et al (J. Lipid. Res. 24, 1127 - 1134 (1983). More specifically, a rabbit is fed with 1% cholesterol food for 3 weeks, dissected, and the small intestine thereof enucleated. The small intestine epithelial is peeled off, crushed by a homogenizer, incomplete debris, etc., removed by centrifugation, and the sediment at 107000 x g as the microsome fraction suspended in 0.154 M phosphate buffer (pH 7.4) to provide an ACAT enzyme preparation. The reaction is carried out by adding 18 nmol of bovine serum albumin and 18 nmol of [1-$^{14}$C]oleylCoA (0.018 $\mu$Ci/nmol) to 0.5 ml of 0.154 M phosphate buffer (pH 7.4), and heating the mixture at 37$^\circ$C for 5 minutes, followed by an addition of 40 $\mu$l of the above ACAT enzyme preparation (0.2 to 0.4 mg as the protein amount). After a predetermined reaction time, 7 ml of dichloromethane:methanol (2:1) mixture is added thereto and thoroughly mixed, the reaction is then stopped simultaneously with collection of the dichloromethane layer, the same fraction is subjected to a separation of the cholesterol oleate portion thereof by silica gel TLC (eluent:petroleum ether:diethyl ether 95:5), and this portion is scraped off and the radioactivity thereof measured by a liquid scintillation counter, whereby the ACAT activity is calculated.

## PREPARATION OF MICROORGANISM LIPID

For example, after culturing the bacteria in a GAM medium (Nissui Seiyaku), the cells are collected by centrifugation (8000 x g), and after washing twice with physiological saline, freeze-dried or dried by heating, subjected to extraction with hexane, ethanol or dichloromethane:methanol (2:1), followed by removal of the solvent by rotary evaporator, to give the lipid.

As the yeast, commercially available baker's yeast is dried and subjected to the same treatment with the use of the above organic solvent.

Lipids can be similarly obtained from rice bran and wheat bran.

## PREPARATION OF LIPID DECOMPOSED PRODUCT

(1) An amount of 1 g of the lipid obtained as described above is suspended in 100 ml of a 0.15 M phosphate buffer (pH 8.0) containing 1% polyvinyl alcohol, and 1% pancreatin, lipase or esterase is added to carry out the treatment at 40$^\circ$C for 3 hours (in place of the lipid obtained as described above, microorganism cells or rice bran or wheat bran also may be directly enzyme-treated). The product is then subjected twice to extraction with hexane of the same volume, the hexane layer is removed, and after the removal of the hexane, the lipid decomposed product is obtained.

(2) To 1 g of the above lipid is added 100 ml of 4N KOH solution, the mixture is stirred to carry out the treatment at 60$^\circ$C for 30 minutes, and then the mixture is made acidic with HCl, extracted with the same volume of hexane or ether, the extracted layer is removed, and after the removal of the organic solvent, the lipid decomposed product is obtained.

## EXAMPLES

The present invention is described below in more detail with reference to Examples, which in no way limit the scope of the present invention.

(1) ACAT inhibitory activity of fatty acid-like substance A (C$_{18}$H$_{13}$2O$_2$) and (C$_{18}$H$_{30}$O$_2$)

(1) In test tube:

| Concentration | Inhibitory ratio (%) | |
|---|---|---|
| (μg/ml) | A | B |
| 400 | 94 | 96 |
| 160 | 74 | 72 |
| 40 | 38 | 46 |
| 10 | 24 | 32 |

(2) Animal test:

To hamsters (male 100 g) were administered the present substance mixed in fodder in an amount of 1 mg per hamster per day, and the ACAT activity of the intestine epithelial cells after one and two weeks was measured.

| ACAT inhibitory ratio (%) | After one week | 55 |
|---|---|---|
| | After two weeks | 49 |

(2) ACAT inhibitory activity of various microorganism lipids and rice bran/wheat bran decomposed products prepared according to the above method

The ACAT activities when 1000 μg/ml of the above-described lipid was added are shown in the following Table.

| Control (not added) | | 100 (%) |
|---|---|---|
| L. reuteri | ATCC 9338 | 29 |
| Bif. bifidum | ATCC 29521 | 7 |
| Bif. longum | FERM P-8562 | 14 |
| Bif. breve | FERM P-8573 | 56 |
| L. bulgaricus | IAM 1120 | 58 |
| L. acidophilus | ATCC 4356 | 48 |
| E. faecalis | FERM BP-297 | 35 |
| Rice bran | lipid enzyme decomposed product | 3 |
| Wheat bran | " | 5 |
| S. cereviciae (yeast) | AHU 3035 | 42 |
| B. subtilis (B. natto) | ATCC 6051 | 48 |
| St. salivarius | FERM BP-299 | 31 |

(3) Dependency of dose

The concentration dependency of the ACAT inhibitory activities of lipids of L. reuteri AD0002, B. bifidum AD00020, and rice bran are shown below.

| Concentration | 125 | 250 | 500 | 1000 μg/mg |
|---|---|---|---|---|
| L. reuteri ATCC | 66* | 53 | 41 | 29 |
| B. bifidum ATCC | 52 | 40 | 23 | 7 |
| Rice bran | 100 | 83 | 48 | 3 |

UTILIZATION IN INDUSTRY

As apparent from the above description, even though the fatty acid-like substance, which is the active ingredient of the ACAT enzyme inhibitory composition according to the present invention, is derived from a food component, and therefore is substantially orally nontoxic, it has an effective ACAT enzyme inhibitory activity and is particularly useful as a functional food component.

**Claims**

1. An ACAT enzyme inhibitory composition which is derived from a microorganism or vegetable lipid and contains a lipid acid-like substance having a molecular formula $C_{18}H_{32}O_2$ or $C_{18}H_{30}O_2$ as the active ingredient.

* Relative activity when Control is made 100.

2. A composition as claimed in claim 1, wherein said fatty acid-like substance is derived from a lactic acid bacterium.

3. A composition as claimed in claim 1, wherein said fatty acid-like substance is derived from rice bran or wheat bran lipid.

# Fig.1

# Fig.2

EP 0 462 291 A1

# Fig.3

# Fig.4

EP 0 462 291 A1

**I. CLASSIFICATION OF SUBJECT MATTER** (if several classification symbols apply, indicate all)

According to International Patent Classification (IPC) or to both National Classification and IPC

Int. Cl$^5$ A23L1/30, A61K37/22, C07G17/00, C12N9/99

**II. FIELDS SEARCHED**

Minimum Documentation Searched

| Classification System | Classification Symbols |
|---|---|
| IPC | C11C1/00-1/10, C07C57/12, A23L1/30, A61K31/20, C12N9/99 |

Documentation Searched other than Minimum Documentation
to the Extent that such Documents are Included in the Fields Searched

COMPUTER SEARCH (CHEMICAL ABSTRACT, MEDLINE DATABASE)

**III. DOCUMENTS CONSIDERED TO BE RELEVANT**

| Category | Citation of Document, with indication, where appropriate, of the relevant passages | Relevant to Claim No. |
|---|---|---|
| X,Y | JP, A, 60-115522 (Century Laboratories, Inc.), June 22, 1985 (22. 06. 85) & US, A, 4526902 & DE, A, 3438630 | 1-3 |
| X,Y | JP, A, 61-21065 (Akiyoshi Kitano, Mitsubishi Kasei Corp.), January 29, 1986 (29. 01. 86), (Family: none) | 1-3 |
| X,Y | JP, A, 61-158764 (Masao Uchida), July 18, 1986 (18. 07. 86), (Family: none) | 1-3 |
| X,Y | JP, A, 62-93225 (Akiyoshi Kitano, Mitsubishi Kasei Corp.), April 28, 1987 (28. 04. 87), (Family: none) | 1-3 |
| X,Y | JP, A, 63-44843 (Kao Corp.), February 25, 1988 (25. 02. 88), (Family: none) | 1-3 |

* Special categories of cited documents: <sup>10</sup>

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure. use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 25, 1991 (25. 03. 91) | April 8, 1991 (08. 04. 91) |

| International Searching Authority | Signature of Authorized Officer |
|---|---|
| Japanese Patent Office | |

FURTHER INFORMATION CONTINUED FROM THE SECOND SHEET

| | | |
|---|---|---|
| X,Y | JP, A, 63-295698 (Showa Denko K.K.), December 2, 1988 (02. 12. 88), (Family: none) | 1-3 |
| X,Y | JP, A, 1-207257 (Nippon Oil and Fats Co., Ltd., Nippon Rensui K.K.), August 21, 1989 (21. 08. 89), (Family: none) | 1-3 |
| X,Y | JP, A, 1-501308 (Laboratoire Natille Medica), May 11, 1989 (11. 05. 89) & WO, A, 8803406 & EP, A, 266468 | 1-3 |

V.☐ OBSERVATIONS WHERE CERTAIN CLAIMS WERE FOUND UNSEARCHABLE [1]

This international search report has not been established in respect of certain claims under Article 17(2) (a) for the following reasons:

1.☐ Claim numbers          because they relate to subject matter not required to be searched by this Authority, namely:

2.☐ Claim numbers          because they relate to parts of the international application that do not comply with the prescribed requirements to such an extent that no meaningful international search can be carried out, specifically:

3.☐ Claim numbers          because they are dependent claims and are not drafted in accordance with the second and third sentences of PCT Rule 6.4(a).

VI.☐ OBSERVATIONS WHERE UNITY OF INVENTION IS LACKING [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for. which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)

| FURTHER INFORMATION CONTINUED FROM THE SECOND SHEET | | |
|---|---|---|
| X,Y | J. OIL. TECHNOL. INDIA. Vol. 4, Nos.Oct - Dec, (1987) S. K. MUKHOPDHYAY et al. [Lipofrac fractionation of rice bran oil - fatty acid], p. 85 - 86 | 1-3 |
| X,Y | ARTERY, Vol. 8, No. 2, (1980) J. V. Turek et al. [EFFECT OF LOW CHOLESTEROL, LINOLEIC ACID ENRICHED DIET ON THROMBOTIC TENDENCY AND PLASMA LIPOPROTEINS IN PATIENTS WITH ANGINA PECTORIS], p. 134 - 139 | 1-3 |

V.☐ OBSERVATIONS WHERE CERTAIN CLAIMS WERE FOUND UNSEARCHABLE [1]

This international search report has not been established in respect of certain claims under Article 17(2) (a) for the following reasons:

1.☐ Claim numbers          , because they relate to subject matter not required to be searched by this Authority, namely:

2.☐ Claim numbers          , because they relate to parts of the international application that do not comply with the prescribed requirements to such an extent that no meaningful international search can be carried out, specifically:

3.☐ Claim numbers          , because they are dependent claims and are not drafted in accordance with the second and third sentences of PCT Rule 6.4(a).

VI.☐ OBSERVATIONS WHERE UNITY OF INVENTION IS LACKING [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)

FURTHER INFORMATION CONTINUED FROM THE SECOND SHEET

| | | |
|---|---|---|
| X,Y | Applied Microbiology and Biotechnology, Vol. 30, No. 3, (1989) Prashant Mishra et al. [Relationship between ethanol tolerance and fatty acyl composition of Saccharomyces cerevisiae], p. 249 - 298 | 1-3 |
| X,Y | Plant Cell Physiol., Vol. 29, No. 4, (1988) Shin - Ichi Toriyama et al. [Prominent Difference of Glycelolipids among Anther Walls, Pollen Grains and Leaves of Rice and Maize], p. 615 - 621 | 1-3 |

V.☐ OBSERVATIONS WHERE CERTAIN CLAIMS WERE FOUND UNSEARCHABLE [1]

This international search report has not been established in respect of certain claims under Article 17(2) (a) for the following reasons:

1.☐ Claim numbers       . because they relate to subject matter not required to be searched by this Authority, namely:

2.☐ Claim numbers       . because they relate to parts of the international application that do not comply with the prescribed requirements to such an extent that no meaningful international search can be carried out, specifically:

3.☐ Claim numbers       . because they are dependent claims and are not drafted in accordance with the second and third sentences of PCT Rule 6.4(a).

VI.☐ OBSERVATIONS WHERE UNITY OF INVENTION IS LACKING [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

| FURTHER INFORMATION CONTINUED FROM THE SECOND SHEET | | |
|---|---|---|
| X,Y | American Journal of Clinical Nutrition, Vol. 17, (1965) D. M. Hegsted et al. [ Quantitative Effects of Dietary Fat on Serum Cholesterol in Man], p. 281 - 295 | 1-3 |
| X,Y | LIPIDS, Vol. 24, No. 4, (1989) M. L. Garg et al. [Dietary Saturated Fat Level Alters the Competition Between α - Linolenic and Linoleic Acid ], p. 334 - 339 | 1-3 |
| X,Y | System. Appl. Microbiol., Vol. 10, No. 2, (1988) | 1-3 |

## V☐ OBSERVATIONS WHERE CERTAIN CLAIMS WERE FOUND UNSEARCHABLE [1]

This international search report has not been established in respect of certain claims under Article 17(2) (a) for the following reasons:

1.☐ Claim numbers , because they relate to subject matter not required to be searched by this Authority, namely:

2.☐ Claim numbers , because they relate to parts of the international application that do not comply with the prescribed requirements to such an extent that no meaningful international search can be carried out. specifically:

3.☐ Claim numbers , because they are dependent claims and are not drafted in accordance with the second and third sentences of PCT Rule 6.4(a).

## VI.☐ OBSERVATIONS WHERE UNITY OF INVENTION IS LACKING [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by·claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

**FURTHER INFORMATION CONTINUED FROM THE SECOND SHEET**

|  | B. C. VILJOEN et al.<br>[ The Significance of Long – Chain Fatty<br>Acid Composition and Other Phenotype<br>Characteristics in Determining Relation-<br>ships Among Some Candida, Kluyveromyces<br>and Saccharomyces Species ], p. 116 – 120 |  |
|---|---|---|
| X,Y | Atherosclerosis, Vol. 66, Nos. 1 – 2, (1987).<br>D. Kromhouto et al.<br>[ Long – term effects of a linoleic acid –<br>enriched diet, changes in body weight and<br>alcohol consumption on serum total and<br>HDL – cholesterol ], p. 99 – 105 | 1-3 |

---

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers     . because they relate to subject matter not required to be searched by this Authority, namely:

2.☐ Claim numbers     . because they relate to parts of the international application that do not comply with the prescribed requirements to such an extent that no meaningful international search can be carried out. specifically:

3.☐ Claim numbers     . because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant. this international search report covers all searchable claims of the international application.

2.☐ As only some of the required additional search fees were timely paid by the applicant, this international search report covers only those claims of the international application for which fees were paid: specifically claims:

3.☐ No required additional search fees were timely paid by the applicant. Consequently, this international search report is restricted to the invention first mentioned in the claims: it is covered by claim numbers:

4.☐ As all searchable claims could be searched without effort justifying an additional fee. the International Searching Authority did not invite payment of any additional fee.

**Remark on Protest**

☐ The additional search fees were accompanied by applicant's protest.

☐ No protest accompanied the payment of additional search fees.

Form PCT/ISA/210 (supplemental sheet (2)) (January 1985)

**FURTHER INFORMATION CONTINUED FROM THE SECOND SHEET**

| X,Y | Acta Paediatr Scand, Vol. 70, No. 5, (1981)<br>J. Fernandes et al.<br>[ THE EFFECT OF A VIRTUALLY CHOLESTEROL<br>FREE, HIGH - LINOLEIC - ACID VEGETARIAN<br>DIET ON SERUM LIPOPROTEINS OF CHILDREN<br>WITH FAMILIAL HYPERCHOLESTEROLEMIA<br>(TYPE II - A)], p. 677 - 682 | 1-3 |
|---|---|---|
| X,Y | Prog. Lipid Res., Vol. 20, (1981)<br>H. M. SINCLAIR<br>[ THE RELATIVE IMPOTANCE OF ESSENTIAL<br>LINOLENIC FAMILIES : STUDIES WITH AN<br>ESKIMO DIET], p. 897 - 899 | 1-3 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers          , because they relate to subject matter not required to be searched by this Authority, namely:

2.☐ Claim numbers          , because they relate to parts of the international application that do not comply with the prescribed requirements to such an extent that no meaningful international search can be carried out, specifically:

3.☐ Claim numbers          , because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

**Remark on Protest**

☐ The additional search fees were accompanied by applicant's protest.

☐ No protest accompanied the payment of additional search fees.

Form PCT/ISA/210 (supplemental sheet (2)) (January 1985)

**FURTHER INFORMATION CONTINUED FROM THE SECOND SHEET**

| | | |
|---|---|---|
| X,Y | The American Journal of Clinical Nutrition, Vol. 32, No. 11, (1979) D. C. Bronsgeest — Schoute et al. [Dependence of the effects of dietary cholesterol and experimental conditions on serum lipids in man], p. 2183 — 2187. | 1-3 |
| X,Y | International Journal of Cardiology, Vol. 17, No. 3, (1987) D. F. Horrobin et al. [The role of linoleic acid and its metabolites in the lowering of plasma cholesterol and the prevention of cardiovascular disease], p. 241 - 255 | 1-3 |

**V.☐ OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers , because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)

FURTHER INFORMATION CONTINUED FROM THE SECOND SHEET

| | |
|---|---|
| X,Y : Journal of Lipid Research, Vol. 27, No. 4, (1986) O. Adam et al. [Effect of α - linolenic acid in the human diet on linoleic acid metabolism and prostaglandin biosymthesis], p. 421 - 426 | 1-3 |

V.☐ OBSERVATIONS WHERE CERTAIN CLAIMS WERE FOUND UNSEARCHABLE [1]

This international search report has not been established in respect of certain claims under Article 17(2) (a) for the following reasons:

1.☐ Claim numbers . because they relate. to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

VI.☐ OBSERVATIONS WHERE UNITY OF INVENTION IS LACKING [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.